# FASCICULE DE BREVET EUROPEEN

(11) **EP 2 525 701 B1**
(45) Date de publication et mention de la délivrance du brevet: **02.11.2016**
(21) Numéro de dépôt: 11704652.4
(22) Date de dépôt: 18.01.2011
(51) Int. Cl.: A61B 1/00, A61B 1/015

(54) **ENDOSCOPE MEDICAL COMPORTANT UN INSTRUMENT CONSOMMABLE AVEC CIRCUIT DE CIRCULATION D'UN FLUIDE**
MEDIZINISCHES ENDOSKOP MIT EINEM VERBRAUCHSINSTRUMENT MIT FLÜSSIGKEITSKREIS
MEDICAL ENDOSCOPE COMPRISING A CONSUMABLE INSTRUMENT HAVING A FLUID FLOW CIRCUIT

(30) Priorité: 19.01.2010 FR 1050333
(43) Date de publication de la demande: 28.11.2012
(73) Titulaire: Axess Vision Technology, 37000 Tours (FR)
(72) Inventeur: MATHIEU, Nicolas, F-69130 Ecully (FR); FRUCTUS, Olivier, F-37530 Nazelles Negron (FR)
(74) Mandataire: Thibault, Jean-Marc
(86) Numéro de dépôt international: PCT/FR2011/050084
(87) Numéro de publication internationale: WO 2011/089349

(56) Documents cités:
- EP-A1- 1 493 379
- WO-A2-94/08654
- US-A- 5 147 292
- US-A- 5 484 402
- US-A- 5 713 850
- US-A1- 2004 158 203
- US-B1- 6 364 853

## Description

La présente invention concerne le domaine technique des appareillages médicaux au sens général et elle vise plus précisément un endoscope médical au sens général permettant d'accéder à l'intérieur d'un corps comme une cavité ou un canal par exemple.

La présente invention concerne plus précisément mais non exclusivement un endoscope médical à usage unique comportant un support d'actionnement tel une poignée destinée à être réutilisée alors que l'instrument médical qui est en contact avec les tissus ou organes humains relève d'un usage unique, voire multiple après décontamination.

L'objet de l'invention trouve des applications particulièrement avantageuses pour permettre d'accéder à la surface interne d'un organe creux, d'une cavité ou d'un conduit naturel ou artificiel du corps humain en vue d'effectuer diverses opérations à des fins thérapeutiques, chirurgicales ou de diagnostic.

L'endoscope selon l'invention est utilisé à des fins de diagnostic, thérapeutiques ou de chirurgie pour l'inspection de toutes les parties internes du corps humain accessibles par les voies naturelles ou artificielles. Par exemple, l'endoscope selon l'invention peut être utilisé dans le domaine des voies urinaires, des voies gastro-intestinales, du système respiratoire, du système cardiovasculaire, de la trachée, de la cavité du sinus, du système de reproduction de la femme, de la cavité abdominale ou de tout autre partie du corps humain à explorer par une voie naturelle ou artificielle.

D'une manière générale, un endoscope médical à usage unique ou patient unique, comporte une poignée de commande à laquelle est assemblé temporairement à l'aide d'un système d'assemblage, un instrument médical consommable se présentant généralement sous la forme d'un tube. Un tel endoscope comporte généralement un système de visualisation optique permettant d'éclairer et d'examiner l'organe, la cavité ou le conduit du corps humain. De manière classique, un endoscope médical comporte également un système pour permettre le pliage ou le béquillage de l'extrémité distale de l'instrument médical afin d'orienter le tube.

Dans l'état de la technique, il est connu diverses solutions d'endoscope comportant un instrument tubulaire séparable par rapport à une poignée. Par exemple, la demande de brevet WO 2008/086497 décrit un endoscope comportant une poignée de support sur laquelle est destiné à être monté temporairement un instrument séparable dont l'extrémité proximale du tube d'insertion est pourvue d'un boîtier de raccordement à l'extrémité de laquelle fait saillie une roue dentée apte à coopérer avec un pignon d'actionnement équipant la poignée. L'assemblage de l'instrument séparable avec la poignée assure la connexion mécanique entre la roue dentée et le pignon. La roue dentée de l'instrument séparable permet d'agir sur des câbles fixés à l'extrémité libre du tube d'insertion pour permettre son orientation. Le boîtier de raccordement comporte également une partie optique permettant de coopérer avec une partie optique complémentaire présentée par la poignée de manière à constituer ensemble, un système de visualisation lorsque l'instrument médical est assemblé sur la poignée.

Dans de nombreuses applications, il apparaît avantageux d'amener au niveau de la partie distale du tube d'insertion, un ou plusieurs appareillages adaptés pour permettre de réaliser différentes fonctions tels que l'amenée de fluide, l'aspiration de fluide, l'amenée d'instruments, effectuer des prélèvements ou des opérations de chirurgie. Pour satisfaire un tel besoin, le brevet US 6 017 322 propose de réaliser un ou plusieurs conduits tubulaires s'étendant le long du tube d'insertion et pourvu à son extrémité proximale d'un raccord amovible de montage sur la poignée. Ce raccord de montage est destiné à être relié à un système pour amener des fluides ou pour aspirer des fluides. Cette solution présente l'avantage que le circuit de circulation de fluide est indépendant de la poignée de sorte que la poignée n'est pas souillée par le fluide provenant du patient. Toutefois, un inconvénient de cet endoscope concerne la difficulté et le temps de montage sur la poignée, des conduits tubulaires et du système de pliage de l'instrument médical. Par ailleurs, cet endoscope n'intègre pas une solution technique pour contrôler, simultanément ou non, l'obturation des conduits tubulaires et par suite, ne permet pas de garantir le maintien d'un circuit stérile jusqu'à son utilisation.

Dans l'état de la technique, il est également connu, différents systèmes permettant de contrôler l'irrigation d'un site chirurgical. Par exemple, les brevets US 5 484 402 et US 6 364 853 décrivent un système d'irrigation et d'aspiration conçu pour être utilisé en association avec un endoscope. Un tel système comporte notamment une poignée de préhension formée par l'assemblage de deux demi-coques à l'intérieur desquelles sont montés un tube d'aspiration et un tube d'injection déformables par l'intermédiaire d'un mécanisme actionné manuellement pour contrôler l'ouverture et la fermeture des tubes. Un tel système est apte à contrôler l'irrigation d'un site chirurgical sans toutefois assurer les fonctions d'un endoscope. Par ailleurs, un tel système n'est pas conçu pour comporter d'une part, une partie réutilisable non souillée au cours de son utilisation et d'autre part, une partie consommable apte à être enlevée facilement par rapport à la partie réutilisable.

La présente invention vise donc à remédier aux inconvénients de l'état de la technique en proposant un nouvel endoscope médical à usage unique, ou patient unique comportant un instrument médical consommable assemblé temporairement avec une poignée de commande, cet endoscope étant conçu pour permettre le montage simple et rapide d'une part, du système pour assurer le pliage ou le béquillage de la tête de l'instrument médical et d'autre part, d'au moins un circuit de circulation d'un fluide, dont l'obturation peut être contrôlée de manière simple et efficace, permettant en particulier, de maintenir stérile ledit circuit de circulation même après son assemblage avec la poignée de commande.

Pour atteindre un tel objectif, l'endoscope médical selon l'invention comporte une poignée de commande pourvue d'un mécanisme de commande et à laquelle est assemblé temporairement à l'aide d'un système d'assemblage, un instrument médical consommable comportant :
- au moins un circuit de circulation d'un fluide dont une partie s'étend le long d'un tube d'insertion, le circuit de circulation comportant un raccord accessible indépendant de la poignée,
- un système pour actionner la tête du tube d'insertion, ce système d'actionnement étant piloté par le mécanisme de commande après assemblage entre la poignée et l'instrument médical consommable.

Selon l'invention, la poignée de commande et l'instrument médical consommable comportent une première partie et une deuxième partie d'un dispositif d'obturation, coopérant entre elles en position assemblée, pour assurer une obturation contrôlée d'au moins un circuit de circulation de fluide.

L'endoscope selon l'invention comporte également en combinaison l'une et/ou l'autre des caractéristiques suivantes :
- le dispositif d'obturation contrôlée assure d'une part la non obturation d'un circuit de circulation avant l'assemblage et d'autre part, l'obturation automatique du circuit de circulation après l'assemblage,
- la première partie et la deuxième partie sont portées respectivement par l'instrument médical consommable et par la poignée, l'une des parties comportant un obturateur tandis que l'autre partie comporte un organe de commande de l'obturateur coopérant avec ledit obturateur après l'assemblage entre la poignée et l'instrument médical consommable,
- la première partie est réalisée par une partie déformable du circuit de circulation, tandis que la deuxième partie comporte un clamp agissant après assemblage, sur la partie déformable du circuit de circulation, et commandé en déplacement par un bouton de commande pour contrôler l'obturation dudit circuit,
- le clamp agit sur au moins deux circuits de circulation de fluide pour simultanément ouvrir l'un et fermer l'autre, et inversement,
- le dispositif d'obturation contrôlée comporte un moyen d'occultation du clamp lors de l'assemblage permettant son positionnement sans déformation de la partie déformable, ce moyen d'occultation n'agissant plus sur le clamp après l'assemblage de sorte que le clamp puisse obturer le circuit de circulation,
- un organe de verrouillage de l'assemblage entre l'instrument médical consommable et la poignée, coopérant automatiquement avec un organe de blocage en fin de course de rapprochement entre le support et l'instrument médical consommable,
- l'organe de blocage est piloté par le bouton de commande du clamp pour assurer le déverrouillage de l'organe de verrouillage conduisant à la séparation entre l'instrument médical consommable et la poignée,
- le système d'actionnement comporte au moins une poulie munie d'un organe d'accouplement mâle ou femelle destiné à coopérer, après assemblage, respectivement avec un organe d'accouplement respectivement femelle ou mâle du mécanisme de commande équipant la poignée,
- le système d'actionnement comporte au moins une paire de poulies montées coaxialement l'une à l'autre,
- chaque organe d'accouplement mâle ou femelle équipant la poignée de commande est pourvu d'un levier de commande,
- l'instrument médical comporte un système de vision apte à éclairer et à ramener une image d'une partie du tube d'insertion éloignée de la partie proximale.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue en perspective d'un endoscope médical conforme à l'invention.
La **Figure 2** est une vue en perspective d'un endoscope médical conforme à l'invention avec l'instrument médical consommable séparé de la poignée.
La **Figure 3** est une vue en perspective montrant l'assemblage de l'instrument médical consommable sur la poignée.
La **Figure 4** est une vue en perspective montrant l'intérieur de l'instrument médical consommable de l'endoscope médical conforme à l'invention.
La **Figure 5** est une vue en perspective illustrant l'intérieur de la poignée de l'endoscope médical conforme à l'invention.
La **Figure 6** est une vue en perspective montrant le clamp mis en oeuvre par l'endoscope conforme à l'invention.
La **Figure 7** est une vue en coupe transversale prise sensiblement selon les lignes A-A de la **Fig. 2** et montrant l'endoscope dans une position avant assemblage.
Les **Figures 7A** et **7B** sont des vues en coupe longitudinale prises sensiblement selon les lignes respectivement VII-A et VII-B de la **Fig. 7****.**
La **Figure 8** est une vue en coupe transversale prise sensiblement selon la ligne A de la **Fig.2** et montrant l'endoscope en position assemblée.
Les **Figures 8A** et **8B** sont des vues en coupe longitudinale prises respectivement selon les lignes VIII-A et VIII-B de la **Fig. 8****.**

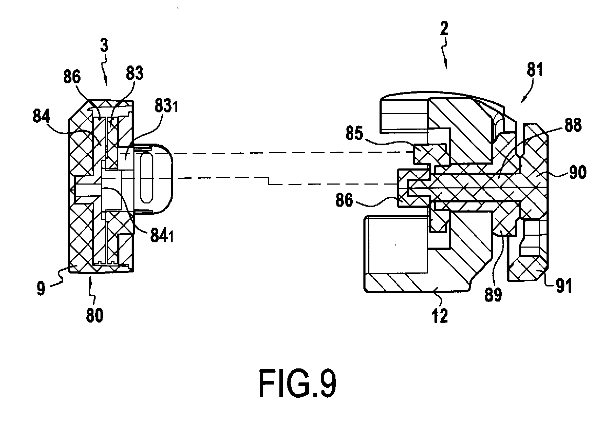

La **Figure 9** montre un exemple de réalisation du système d'actionnement du tube d'insertion.

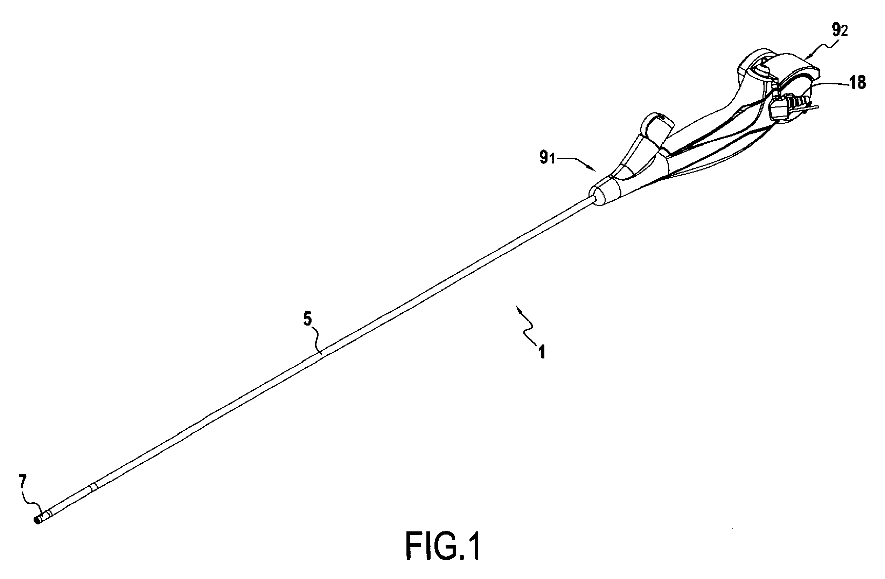

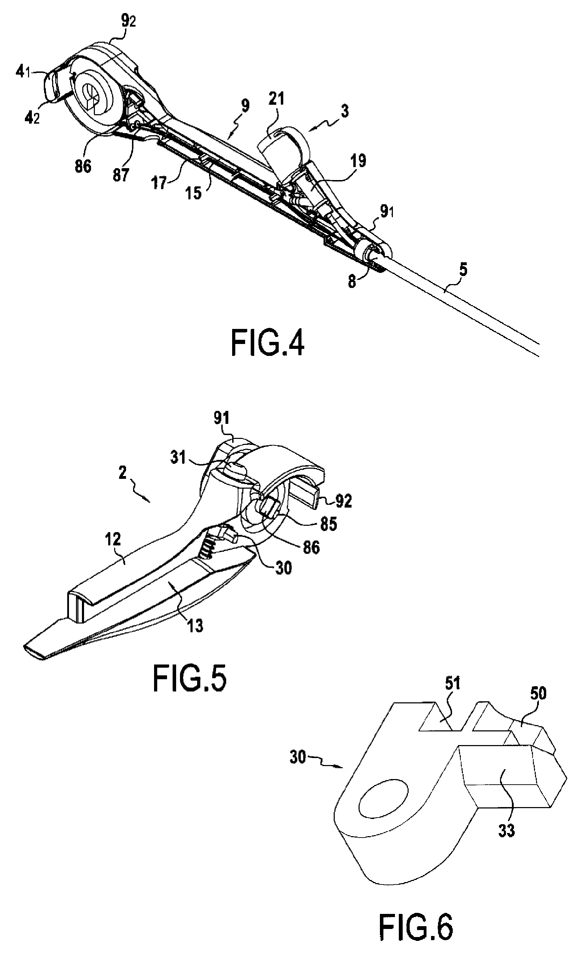

Tel que cela ressort plus précisément des **Fig. 1** et **5****,** l'objet de l'invention concerne un endoscope médical **1** comportant un support d'actionnement **2** tel qu'une poignée de commande sur laquelle est assemblé temporairement un instrument médical consommable **3.** Cet instrument médical consommable **3** qui est en contact avec les tissus ou les organes humains relèvent essentiellement d'un usage unique ou multiple d'un patient voire d'un usage réutilisable après décontamination, désinfection et voire une stérilisation.

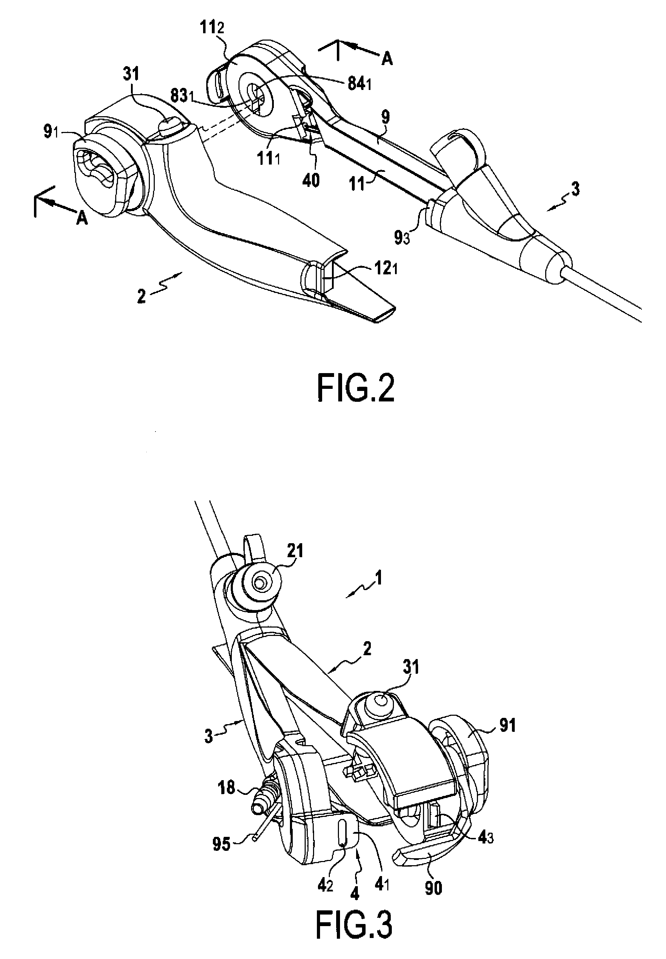

L'instrument médical consommable **3** est destiné à être assemblé de façon temporaire au support **2** à l'aide d'un système d'assemblage **4.** Selon un exemple avantageux de réalisation, le système d'assemblage **4** est du type à encliquetage comme cela sera expliqué dans la suite de la description. Le système d'assemblage **4** est adapté pour assurer rapidement une liaison au moins mécanique temporaire tout en offrant l'avantage de permettre une séparation facile de l'instrument médical consommable **3** par rapport au support **2.** Le système d'assemblage **4** assure ainsi une liaison démontable entre la poignée de commande **2** et l'instrument médical consommable **3** permettant de pouvoir réutiliser la poignée de commande **2** avec d'autres instruments médicaux consommables **3.** La **Fig. 1** illustre l'endoscope en position assemblée tandis que la **Fig. 2** illustre l'instrument médical consommable **3** séparé par rapport au support **2.** Cette dernière position de l'endoscope sera désignée dans la suite de la description par position séparée.

De manière classique, l'instrument médical consommable **3** comporte un tube d'insertion externe **5** présentant une longueur et une flexibilité plus ou moins importante et destiné à être introduit dans une voie d'accès naturel ou artificiel en vue d'effectuer diverses opérations ou fonctions à des fins thérapeutiques, chirurgicales ou de diagnostic. Par exemple, le tube d'insertion **5** possède une partie distale **7** formant la tête de l'endoscope **1.** Le tube d'insertion **5** comporte également une partie proximale **8** opposée à la partie distale **7** et s'étendant en saillie à partir de l'extrémité distale **9₁** d'un boîtier **9** formant le corps principal de montage sur le support **2.**

Ce boîtier **9** se présente sous la forme d'un corps allongé de section droite transversale sensiblement en forme de « U » dont la face ouverte est fermée par un couvercle **11.** De même, le support **2** se présente sous la forme d'un corps allongé **12** présentant une section droite en forme de « U » dont la face ouverte **13** est destinée à venir en correspondance avec le couvercle **11** lors de l'assemblage du support **2** avec l'instrument médical consommable **3.** Il doit être considéré qu'en position assemblée, le support **2** et le boîtier **9** de l'instrument médical consommable **3** forment ensemble une poignée de préhension permettant une préhension aisée de l'endoscope. En d'autres termes, le corps **12** et le boîtier **9** forment chacun une demi-coque qui, après assemblage, réalise la forme définitive de la poignée.

L'instrument médical consommable **3** comporte au moins un circuit **15** de circulation d'un fluide s'étendant le long du tube d'insertion **5** entre les extrémités distale **7** et proximale **8** (**Fig. 4**). Dans l'exemple illustré, le circuit de circulation **15** comporte un conduit tubulaire s'étendant à l'intérieur du tube d'insertion **5** jusqu'à sa partie distale **7.** Ce conduit tubulaire **15** sort du tube d'insertion **5** en se prolongeant à l'intérieur du boîtier **9.** Avantageusement, le conduit tubulaire **15** est maintenu en position fixe à l'intérieur du boîtier par des pattes **17** s'étendant à l'intérieur du boîtier. Le conduit tubulaire **15** sort à la partie proximale **9₂** du boîtier opposé de la partie distale **9₁** (**Fig. 3**). Le conduit tubulaire **15** est muni à l'extérieur du boîtier **9,** d'un raccord **18** à une source d'aspiration ou d'amenée d'un fluide.

Selon une variante avantageuse de réalisation, le circuit de circulation **15** comporte une branche de dérivation **19** de sorte que le circuit forme en cet endroit un « Y ». Dans l'exemple de réalisation illustré, la branche de dérivation **19** est réalisée au niveau de l'extrémité distale **9₁** du boîtier **9** qui constitue ainsi un nez de forme triangulaire. Cette branche de dérivation **19** sert au passage d'outillages divers destinés à être amenés à la partie distale **7** du tube d'insertion **5.** Cette branche de dérivation **19** est fermée éventuellement par un bouchon **21.**

Conformément à l'invention, la poignée de commande **2** et l'instrument médical consommable **3** comportent respectivement une première partie et une deuxième partie d'un dispositif d'obturation coopérant entre elles en position assemblée pour assurer une obturation contrôlée pour au moins le circuit de circulation **15.**

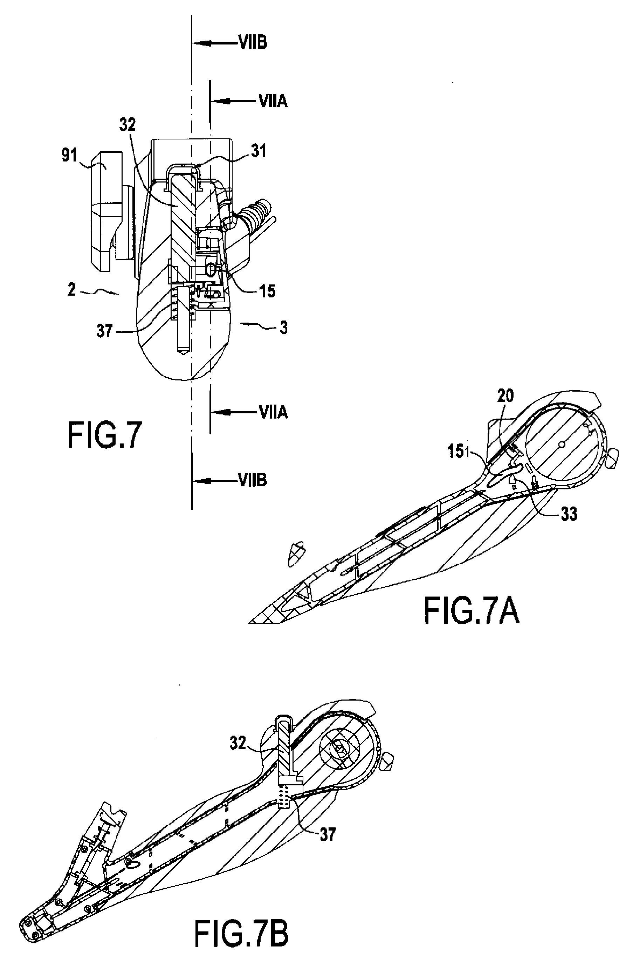

Dans l'exemple de réalisation illustré, la première partie équipe l'instrument médical consommable **3** et se trouve réalisée par une partie déformable **15₁** du circuit de circulation **15** (**Fig. 7, 7A**). Avantageusement, cette partie déformable **15₁** est maintenue en place, dans le boitier **9,** par des butées de positionnement **20.**

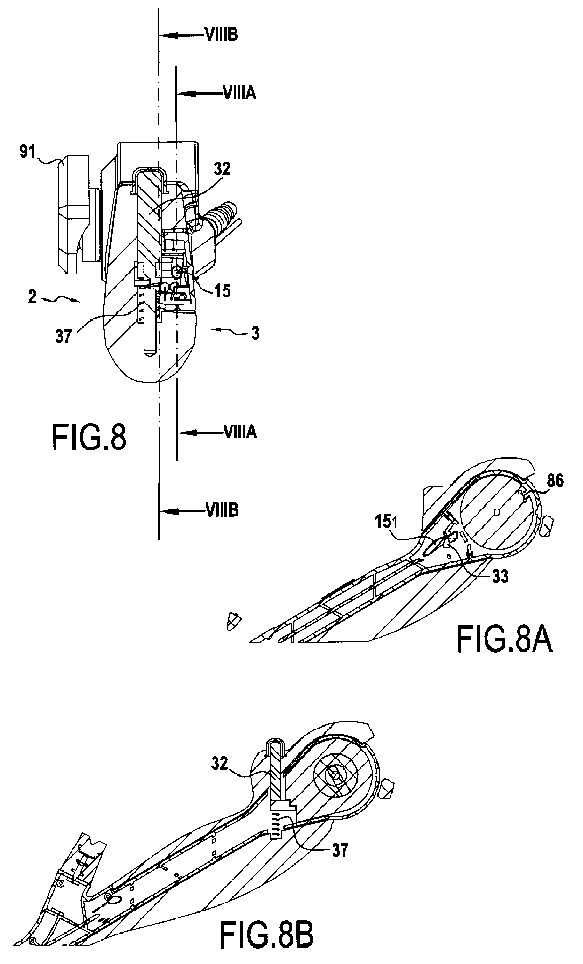

La deuxième partie du dispositif d'obturation contrôlée est portée par le support de commande **2** et comporte un obturateur ou un clamp **30** agissant après assemblage sur la partie déformable **15₁** du circuit. Ce clamp **30** est commandé en déplacement par l'intermédiaire d'un bouton de commande **31** permettant de contrôler l'obturation du circuit **15.** Dans l'exemple illustré, le clamp **30** est monté mobile linéairement sensiblement selon une direction transversale à la partie déformable **15₁** du circuit de fluide pour permettre son pincement (**Fig. 7A**, **8A**). Le clamp **30** présente à cet effet une partie saillante **33** biseautée adaptée pour agir sur une paroi du circuit **15₁.** Avantageusement, la partie déformable du circuit **15** opposée à celle sur laquelle appuie la partie saillante **33,** se trouve en appui sur une butée **20** afin de garantir le contrôle de l'obturation du circuit **15.**

Tel que cela ressort plus précisément de la **Fig. 7****,** le clamp **30** est porté par un axe **32** coulissant faisant saillie du support **2** pour former le bouton de commande **31.** L'axe **32** est sollicité par un ressort **37** permettant, en l'absence d'un effort de pression sur le bouton de commande **31,** de placer le clamp **30** dans une position d'obturation complète du circuit **15** c'est-à-dire de pincement de la partie déformable **15₁** du circuit par la partie saillante **33** (**Fig. 8, 8A, 8B**).

Tel que cela ressort plus précisément de la **Fig. 2****,** il est à noter que le couvercle **11** comporte une ouverture **40** permettant le passage du clamp **30** lors de l'opération d'assemblage et son positionnement par rapport à la partie déformable **15₁** du circuit.

Selon une caractéristique avantageuse de réalisation, le dispositif d'obturation contrôlée comporte un moyen d'occultation **50** du clamp **30** lors de l'assemblage permettant la coopération entre le clamp et le circuit **15** sans déformation de la partie déformable **15₁** du circuit. Ce moyen d'occultation **50** est formé par une rampe réalisée sur le clamp **30** et sur laquelle vient en appui lors de l'opération d'assemblage, le bord **11₁** d'un couvercle auxiliaire **11₂** venant s'établir dans le même plan que le couvercle **11.** Le bord **11₁** du couvercle auxiliaire **11₂** délimite en partie l'ouverture **40.** Ainsi, lors du rapprochement du support **2** et de l'instrument médical consommable **3,** le bord **11₁** du couvercle auxiliaire **11₂** vient en appui sur la rampe **50** entraînant le coulissement du clamp **30** en direction de sa position de non obturation du circuit. Lors de ce déplacement, le ressort **37** est comprimé. La partie déformable **15₁** du circuit vient alors s'établir dans l'espace ainsi dégagé par le clamp **30.** Ainsi, le rapprochement de l'instrument médical consommable **3** par rapport au support **2** n'entraîne pratiquement aucune déformation de la partie déformable **15₁** du circuit. En fin de course de rapprochement, le bord **11₁** du couvercle auxiliaire **11₂** s'échappe de la rampe **50** pour venir dans un logement **51** de dégagement aménagé sur le clamp **30.** Dans cette position finale d'assemblage, le clamp **30** ne se trouve plus sollicité par le bord **11₁** du couvercle auxiliaire **11₂** de sorte que sous l'action du ressort de compression **37,** le clamp **30** coulisse librement pour venir occuper sa position de pincement ou d'obturation partielle ou totale de la partie déformable **15₁** du circuit (**Fig. 8, 8A, 8B**).

Il découle de la description qui précède que le dispositif d'obturation contrôlée assure la non obturation du circuit **15** avant son assemblage avec le support **2.** Ainsi, le circuit **15** n'est pas déformé pendant toute la durée de stockage de l'instrument médical consommable **3.** Par ailleurs, l'assemblage de l'instrument médical consommable **3** avec le support conduit à l'obturation automatique du circuit **15** par le clamp **30** permettant d'isoler le circuit **15** par rapport à l'extérieur. Le rapprochement entre le support **2** et l'instrument médical consommable **3** permet l'assemblage automatique de ces pièces grâce à la mise en oeuvre du système d'assemblage **4** du type à encliquetage.

Dans l'exemple décrit, le clamp **30** agit sur un seul circuit de circulation **15.** Bien entendu, le clamp **30** peut agir simultanément sur plusieurs circuits de circulation **15.** Par exemple, ce clamp **30** peut être aménagé pour fermer ou ouvrir simultanément les circuits de circulation **15** ou pour simultanément ouvrir au moins un circuit de circulation **15** et fermer au moins un autre circuit de circulation **15,** et inversement.

Selon l'exemple de réalisation illustré, le système d'assemblage **4** comporte une languette d'encliquetage **4₁** s'étendant en saillie à partir par exemple de l'extrémité proximale **9₂** du boîtier. Cette languette **4₁** comporte un orifice **4₂** destiné à s'engager lors du rapprochement entre le support **2** et l'instrument médical consommable **3,** dans un téton **4₃** porté par l'extrémité proximale du support **2.** Par ailleurs, le support **2** comporte à son extrémité distale, un bord d'appui **12₁** venant s'engager sous un rebord ou une lèvre **9₃** aménagé(e) sur le boîtier **9.** L'assemblage de l'endoscope **1** consiste comme cela apparaît plus précisément à la **Fig. 3****,** à engager le bord d'appui **12₁** du support **2** en dessous de la lèvre **9₃** du boîtier **9** puis à rapprocher les extrémités proximales du boîtier **9** et du support **12** jusqu'à l'engagement de la languette **4₁** dans le téton d'encliquetage **4₃.**

Dans la description qui précède, le système d'assemblage **4** est du type à encliquetage. Bien entendu, le système d'assemblage **4** peut être de nature différente. Par exemple, le système d'assemblage **4** peut comporter des leviers ou des crochets de fixation assurant un montage et un démontage facile entre la poignée de commande **2** et l'instrument médical consommable **3.**

Selon une caractéristique avantageuse de réalisation, l'endoscope **1** selon l'invention comporte également un organe de verrouillage de l'assemblage entre le support **2** et l'instrument médical consommable **3** afin d'éviter une séparation intempestive ou accidentelle entre ces deux pièces. L'organe de verrouillage est commandé pour autoriser la séparation entre le support **2** et l'instrument médical consommable **3** après utilisation. Dans l'exemple illustré, le verrouillage de l'assemblage est assuré par l'engagement du bord **11₁** du couvercle auxiliaire **11₂** dans le logement **51** du clamp **30.** Comme expliqué ci-dessus, le verrouillage de l'assemblage entre le support **2** et l'instrument médical consommable **3** est réalisé automatiquement lors de l'opération d'assemblage entre le support **2** et l'instrument médical consommable **3** puisque le clamp **30** vient occuper sa position de blocage du bord **11₁** sous l'action du ressort **37.** Ainsi, le bord de verrouillage **11₁** coopère automatiquement avec le logement de blocage **51** en fin de course entre le support **2** et l'instrument médical consommable **3.** Le déverrouillage est obtenu d'une part, par l'actionnement du bouton de commande **31** conduisant au dégagement du bord **11₁** par rapport au logement de blocage **51,** et d'autre part, par un décrochage de la languette d'encliquetage **4₁** par rapport au téton **4₃,** suivi par un mouvement d'écartement entre le support **2** et l'instrument médical consommable **3,** au niveau de la languette **4₁.** L'organe de blocage **51** (tel le logement) est piloté par le bouton de commande **31** du clamp **30** pour assurer le déverrouillage de l'organe de verrouillage **11₁** conduisant à la séparation entre l'instrument médical consommable et la poignée. Cette double action permet ainsi de détacher l'instrument médical consommable **3** par rapport au support **2,** de manière sûre et contrôlée.

Selon une variante préférée de réalisation, l'endoscope médical **1** comporte également un mécanisme permettant d'actionner la tête **7** du tube d'insertion **5.** L'instrument médical consommable **3** comporte ainsi un système **80** pour actionner la tête **7** du tube d'insertion **5.** Ce système d'actionnement **80** est piloté par un mécanisme de commande **81** équipant le support **2.** Selon une variante préférée de réalisation, le système d'actionnement **80** comporte au moins une première poulie et dans l'exemple illustré une première **83** et une deuxième **84** poulies munies chacune d'un organe d'accouplement mâle ou femelle **83₁, 84₁** destiné à coopérer avec un organe d'accouplement mâle ou femelle **85, 86** équipant le support de commande **3.** Les deux poulies **83, 84** sont montées avantageusement coaxialement l'une à l'autre à l'intérieur du boîtier **9.** Chaque poulie **83, 84** est pourvue sur sa face transversale, d'un organe d'accouplement mâle ou femelle **83₁, 84₁.** A la périphérie de chaque poulie **83, 84** est fixée l'extrémité d'au moins un câble **86,** monté à l'intérieur du boîtier **9.** De manière classique et telle que cela ressort précisément de la **Fig. 4****,** chaque câble **86** est monté à l'intérieur d'une gaine **87** montée dans le boîtier **9** et passant à l'intérieur du tube d'insertion **5.** L'extrémité du ou des câbles **86** est reliée à la tête **7** du tube **5.**

Ainsi, l'une des poulies **83, 84** commande par exemple le déplacement gauche - droite de la tête **7** alors que l'autre poulie commande le déplacement bas - haut. Ces poulies **83, 84** sont destinées à être reliées mécaniquement aux organes d'accouplement mâle et femelle **85, 86** qui sont entraînés en rotation par tous moyens manuels ou motorisés. Dans l'exemple illustré, les organes d'accouplement mâle et femelle **85, 86** sont aménagés sur des disques **88, 89** montés coaxialement l'un dans l'autre dans le corps **12.** Ces disques **88, 89** sont pourvus chacun d'un levier de commande **90, 91** permettant de commander le déplacement en rotation des organes d'accouplement mâle et femelle **85, 86** et par suite, des poulies **83, 84.** Dans l'exemple illustré, chaque levier de commande **90, 91** est du type rotatif. L'un des leviers s'étend latéralement par rapport au boîtier **9** tandis que l'autre levier s'étend à l'arrière de la partie distale du boîtier **9.** Bien entendu, les leviers de commande **90, 91** peuvent être de nature différente par exemple linéaire.

Il ressort de la description qui précède que l'assemblage entre la poignée de commande **2** et l'instrument médical consommable **3** conduit à la coopération du mécanisme de commande **81** équipant la poignée **2** avec le système d'actionnement **80** faisant partie de l'instrument médical consommable **3.** Le montage d'un instrument médical consommable **3** sur la poignée de commande **5** permet d'activer d'une part la fonction de contrôle de la fermeture/ouverture du circuit de circulation de fluide **15** et d'autre part, la fonction d'actionnement de la tête du tube d'insertion **5.**

L'endoscope **1** conforme à l'invention comporte également un système de vision **95** apte à éclairer et à ramener une image d'une partie du tube d'insertion **5,** éloignée de la partie proximale du tube d'insertion **5.** Par exemple, le système de vision **95** est apte à éclairer et à ramener une image de la partie distale **7** du tube d'insertion **5.** L'instrument médical consommable **3** comporte ainsi un moyen de vision monté à l'intérieur du boîtier **9** et pénétrant à l'intérieur du tube **5** jusqu'à la tête **7** du tube d'insertion **5.**

L'invention n'est pas limitée aux exemples décrits et représentés car diverses modifications peuvent y être apportées sans sortir de son cadre.

## Revendications

1. Endoscope médical comportant une poignée de commande (**2**) pourvue d'un mécanisme de commande (**81**) et à laquelle est assemblé temporairement à l'aide d'un système d'assemblage (**4**), un instrument médical consommable (**3**) comportant :
- au moins un circuit de circulation (**15**) d'un fluide dont une partie s'étend le long d'un tube d'insertion (**5**), le circuit de circulation comportant un raccord (**18**) accessible indépendant de la poignée,
- un système (**80**) pour actionner la tête (**7**) du tube d'insertion (**5**), ce système d'actionnement étant piloté par le mécanisme de commande (**81**) après assemblage entre la poignée (**2**) et l'instrument médical consommable (**3**),
la poignée de commande (**2**) et l'instrument médical consommable (**3**) comportant une première partie et une deuxième partie d'un dispositif d'obturation, coopérant entre elles en position assemblée, pour assurer une obturation contrôlée d'au moins le circuit de circulation de fluide (**15**), **caractérisé en ce que** la première partie est réalisée par une partie déformable (**15₁**) du circuit de circulation, tandis que la deuxième partie comporte un clamp (**30**) agissant après assemblage, sur la partie déformable (**15₁**) du circuit de circulation pour assurer l'obturation automatique du circuit de circulation (**15**), et commandé en déplacement par un bouton de commande (**31**) pour contrôler l'obturation dudit circuit.

2. Endoscope médical selon la revendication 1, **caractérisé en ce que** la première partie du dispositif d'obturation est réalisée par une partie déformable (**15₁**) du circuit de circulation (**15**), non obturée avant assemblage.

3. - Endoscope médical selon la revendication 1 ou 2, **caractérisé en ce que** la première partie et la deuxième partie sont portées respectivement par l'instrument médical consommable (**3**) et par la poignée (**2**), l'une des parties comportant un obturateur tandis que l'autre partie comporte un organe de commande de l'obturateur coopérant avec ledit obturateur après l'assemblage entre la poignée (**2**) et l'instrument médical consommable (**3**).

4. Endoscope médical selon la revendication 1, **caractérisé en ce que** l'instrument médical consommable (**3**) comporte deux circuits de circulation (**15**) et **en ce que** le clamp (**30**) agit sur les deux circuits de circulation de fluide pour simultanément ouvrir l'un et fermer l'autre, et inversement.

5. Endoscope médical selon la revendication 4, **caractérisé en ce que** le dispositif d'obturation contrôlée comporte un moyen d'occultation (**50**) du clamp (**30**) lors de l'assemblage permettant son positionnement sans déformation de la partie déformable (**15₁**), ce moyen d'occultation (**50**) n'agissant plus sur le clamp (**30**) après l'assemblage de sorte que le clamp puisse obturer le circuit de circulation (**15**).

6. Endoscope médical selon l'une des revendications 1 à 5, **caractérisé en ce qu'**il comporte un organe de verrouillage (**11₁**) de assemblage entre l'instrument médical consommable (**3**) et la poignée (**2**), coopérant automatiquement avec un organe de blocage (**51**) en fin de course de rapprochement entre le support (**2**) et l'instrument médical consommable (**3**).

7. Endoscope médical selon la revendication 6, **caractérisé en ce que** l'organe de blocage (**51**) est piloté par le bouton de commande (**31**) du clamp (**30**) pour assurer le déverrouillage de l'organe de verrouillage (**11₁**) conduisant à la séparation entre l'instrument médical consommable et la poignée.

8. Endoscope médical selon la revendication 1, **caractérisé en ce que** le système d'actionnement (**80**) comporte au moins une poulie (**83, 84**) munie d'un organe d'accouplement mâle ou femelle **(83₁, 84₁**) destiné à coopérer, après assemblage, respectivement avec un organe d'accouplement respectivement femelle ou mâle (**85, 86**) du mécanisme de commande (**81**) équipant la poignée (**2**).

9. Endoscope médical selon la revendication 6, **caractérisé en ce que** le système d'actionnement (**80**) comporte au moins une paire de poulies (**83, 84**) montées coaxialement l'une à l'autre.

10. Endoscope médical selon la revendication 8, **caractérisé en ce que** chaque organe d'accouplement mâle ou femelle (**85, 86**) équipant la poignée de commande est pourvu d'un levier de commande (**90, 91**).

11. Endoscope médical selon l'une des revendications 1 à 10, **caractérisé en ce que** l'instrument médical (**1**) comporte un système de vision apte à éclairer et à ramener une image d'une partie (**7**) du tube d'insertion (**5**) éloignée de la partie proximale.

## Patentansprüche

1. Medizinisches Endoskop, das einen Steuergriff (2) aufweist, der mit einem Steuermechanismus (81) versehen ist und an den zeitweilig mit Hilfe eines Verbindungssystems (4) ein medizinisches Verbrauchsinstrument (3) befestigt ist, umfassend:
- mindestens einen Flüssigkeitskreis (15), von dem sich ein Teil entlang eines Einführschlauchs (5) erstreckt, wobei der Flüssigkeitskreis ein zugängliches Anschlussstück (18) aufweist, das von dem Griff unabhängig ist,
- ein System (80) zum Betätigen des Kopfes (7) des Einführschlauchs (5), wobei das System zum Betätigen von dem Steuermechanismus (81) nach dem Zusammenbauen zwischen dem Griff (2) und dem medizinisches Verbrauchsinstrument (3) gesteuert wird,
wobei der Steuergriff (2) und das medizinische Verbrauchsinstrument (3) einen ersten Teil und einen zweiten Teil einer Verschlussvorrichtung aufweisen, die in zusammengebauter Position miteinander zusammenwirken, um ein kontrolliertes Verschließen von mindestens dem Flüssigkeitskreis (15) zu gewährleisten,
**dadurch gekennzeichnet, dass** der erste Teil durch einen verformbaren Teil (15₁) des Flüssigkeitskreises erstellt ist, während der zweite Teil eine Klemme (30) aufweist, die nach dem Zusammenbauen auf den verformbaren Teil (15₁) des Flüssigkeitskreises einwirkt, um das automatische Verschließen des Flüssigkeitskreises (15) zu gewährleisten und die in Verschiebung durch einen Steuerknopf (31) gesteuert wird, um das Verschließen des Kreises zu steuern.

2. Medizinisches Endoskop nach Anspruch 1, **dadurch gekennzeichnet, dass** der erste Teil der Verschlussvorrichtung durch einen verformbaren Teil (15₁) des Flüssigkeitskreises (15) erstellt ist, der vor dem Zusammenbauen nicht verschlossen ist.

3. Medizinisches Endoskop nach Anspruch 1 oder 2, **dadurch gekennzeichnet, dass** der erste Teil und der zweite Teile jeweils von dem medizinischen Verbrauchsinstrument (3) und von dem Griff (2) getragen sind, wobei einer der Teile einen Verschluss aufweist, während der andere Teil ein Steuerorgan des Verschlusses aufweist, das mit dem Verschluss nach dem Zusammenbauen zwischen dem Griff (2) und dem medizinisches Verbrauchsinstrument (3) zusammenwirkt.

4. Medizinisches Endoskop nach Anspruch 1, **dadurch gekennzeichnet, dass** das medizinische Verbrauchsinstrument (3) zwei Flüssigkeitskreise (15) aufweist und dass die Klemme (30) auf die zwei Flüssigkeitskreise einwirkt, um gleichzeitig den einen zu öffnen und den anderen zu schießen und umgekehrt.

5. Medizinisches Endoskop nach Anspruch 4, **dadurch gekennzeichnet, dass** die Vorrichtung zum kontrollierten Verschließen ein Mittel zum Abdecken (50) der Klemme (30) beim Zusammenbauen aufweist, das ihr Positionieren ohne Verformen des verformbaren Teils (15₁) ermöglicht, wobei dieses Mittel zum Abdecken (50) nach dem Zusammenbauen nicht mehr auf die Klemme (30) einwirkt, derart, dass die Klemme den Flüssigkeitskreis (15) verschließen kann.

6. Medizinisches Endoskop nach einem der Ansprüche 1 bis 5, **dadurch gekennzeichnet, dass** es ein Verriegelungsorgan (11₁) der Anordnung zwischen dem medizinischen Verbrauchsinstrument (3) und dem Griff (2) aufweist, das automatisch mit einem Blockierorgan (51) in der Endlage des Annäherns zwischen dem Träger (2) und dem medizinischen Verbrauchsinstrument (3) zusammenwirkt.

7. Medizinisches Endoskop nach Anspruch 6, **dadurch gekennzeichnet, dass** das Blockierorgan (51) durch den Steuerknopf (31) der Klemme (30) gesteuert ist, um das Entriegeln des Verriegelungsorgans (11₁) zu gewährleisten, was zu der Trennung zwischen dem medizinischen Verbrauchsinstrument und dem Griff führt.

8. Medizinisches Endoskop nach Anspruch 1, **dadurch gekennzeichnet, dass** das System zum Betätigen (80) mindestens eine Rolle (83, 84) aufweist, die mit einem Verbindungsorgan zum Einstecken oder zum Aufnehmen (83₁, 84₁) versehen ist, das dazu bestimmt ist, nach dem Zusammenbauen jeweils mit einem Verbindungsorgan zum Einstecken beziehungsweise zum Aufnehmen (85, 86) des Steuermechanismus (81), das den Griff (2) ausstattet, zusammenzuwirken.

9. Medizinisches Endoskop nach Anspruch 6, **dadurch gekennzeichnet, dass** das System zum Betätigen (80) mindestens ein Paar Rollen (83, 84) aufweist, die koaxial zueinander befestigt sind.

10. Medizinisches Endoskop nach Anspruch 8, **dadurch gekennzeichnet, dass** jedes Verbindungsorgan zum Einstecken oder zum Aufnehmen (85, 86), das den Steuergriff ausstattet, mit einem Steuerhebel (90, 91) versehen ist.

11. Medizinisches Endoskop nach einem der Ansprüche 1 bis 10, **dadurch gekennzeichnet, dass** das medizinische Instrument (1) ein Visions-System aufweist, das geeignet ist, ein Bild eines Teils (7) des Einführschlauchs (5), der von dem proximalen Teil entfernt ist, zu beleuchten und wiederzugeben.

## Claims

1. A medical endoscope including a control handle (2) provided with a control mechanism (81) and that, by means of an attachment system (4), is temporarily attached to a consumable medical instrument (3) including:
· at least one flow circuit (15) for a fluid, a portion of which circuit extends along an insertion tube (5), the fluid circuit including a connector fitting (18) accessible independently from the handle; and
· an actuation system (80) for actuating the head (7) of the insertion tube (5), which actuation system is driven by the control mechanism (81) after the handle (2) and the consumable medical instrument (3) have been attached together;
· the control handle (2) and the consumable medical instrument (3) comprising respectively a first portion and a second portion of a closure device, which portions co-operate with each other in the attached position to close off at least the fluid flow circuit (15) in controlled manner,
**characterized in that** the first portion is formed by a deformable portion (15₁) of the flow circuit, while the second portion comprises a clamp (30) acting after attachment and on the deformable portion (15₁) of the flow circuit in order to close off the flow circuit (15) automatically, and caused to move by a control button (31) for controlling closure of said circuit.

2. A medical endoscope according to claim 1, **characterized in that** the first portion of the closure device is formed by a deformable portion (15₁) of the flow circuit (15) that is not closed off prior to attachment.

3. A medical endoscope according to claim 1 or claim 2, **characterized in that** the first portion and the second portion are carried respectively by the consumable medical instrument (3) and by the handle (2), one of the portions comprising a closure member while the other portion comprises a control member for controlling the closure member and co-operating with said closure member after the handle (2) and the consumable medical instrument (3) have been attached together.

4. A medical endoscope according to claim 1, **characterized in that** the consumable medical instrument (3) has two flow circuits (15), and **in that** the clamp (30) acts on both of the fluid flow circuits to open one and to close the other simultaneously, and vice versa.

5. A medical endoscope according to claim 4, **characterized in that** the controlled closure device includes masking means (50) for masking the clamp (30) during the attachment, enabling it to be positioned without deforming the deformable portion (15₁), these masking means (50) no longer acting on the clamp (30) after attachment so that the clamp can close off the flow circuit (15).

6. A medical endoscope according to any one of claims 1 to 5, **characterized in that** it includes a locking member (11₁) for locking the attachment between the consumable medical instrument (3) and the handle (2), which locking member co-operates automatically with a blocking member (51) at the end of the stroke over which the support (2) and the consumable medical instrument (3) are brought together.

7. A medical endoscope according to claim 6, **characterized in that** the blocking member (51) is driven by the control button (31) of the clamp (30) in order to unlock the locking member (11₁) causing the consumable medical instrument and the handle to be detached.

8. A medical endoscope according to claim 1, **characterized in that** the actuation system (80) includes at least one pulley (83, 84) provided with a male or female coupling member (83₁, 84₁) designed to co-operate, after attachment, respectively with a respectively female or male coupling member (85, 86) of the control mechanism (81) equipping the handle (2).

9. A medical endoscope according to claim 6, **characterized in that** the actuation system (80) includes at least one pair of pulleys (83, 84) mounted on a common axis.

10. A medical endoscope according to claim 8, **characterized in that** each male or female coupling member (85, 86) equipping the control handle is provided with a control lever (90, 91).

11. A medical endoscope according to any one of claims 1 to 10, **characterized in that** the medical instrument (1) includes a vision system suitable for illuminating and for returning an image from a portion (7) of the insertion tube (5) that is remote from the proximal portion.
